# EUROPÄISCHE PATENTSCHRIFT

(11) **EP 2 944 686 B1**
(45) Veröffentlichungstag und Bekanntmachung des Hinweises auf die Patenterteilung: **10.10.2018**
(21) Anmeldenummer: 15166505.6
(22) Anmeldetag: 06.05.2015
(51) Int. Cl.: C12M 1/00, B03C 1/01, B03C 1/28, A61M 1/34, A61M 1/36

(54) **VERFAHREN UND VORRICHTUNG ZUR ABREICHERUNG VON ZIRKULIERENDEN TUMORZELLEN AUS EINER ZELLSUSPENSION**
METHOD AND DEVICE FOR THE REMOVAL OF CIRCULATING TUMOR CELLS FROM A CELL SUSPENSION
PROCÉDÉ ET DISPOSITIF D'APPAUVRISSEMENT DE CELLULES TUMORALES CIRCULANTES À PARTIR DE CELLULES EN SUSPENSION

(30) Priorität: 12.05.2014 DE 102014106603
(43) Veröffentlichungstag der Anmeldung: 18.11.2015
(73) Patentinhaber: Verein zur Förderung von Innovationen durch Forschung, Entwicklung und Technologietransfer e.V. (Verein INNOVENT e.V.), 07745 Jena (DE)
(72) Erfinder: Röder, Michael, 07586 Oberndorf (DE); Payer, Petra, 99092 Erfurt (DE)
(74) Vertreter: Oehmke, Volker

(56) Entgegenhaltungen:
- WO-A1-90/04019
- WO-A1-2009/155384
- WO-A2-02/094350
- DE-A1- 10 127 068
- US-A1- 2010 317 093
- CLEMENT J H ET AL: "Differential interaction of magnetic nanoparticles with tumor cells and peripheral blood cells", JOURNAL OF CANCER RESEARCH AND CLINICAL ONCOLOGY, SPRINGER, BERLIN, DE, Bd. 132, Nr. 5, 1. Mai 2006 (2006-05-01), Seiten 287-292, XP019345489, ISSN: 1432-1335, DOI: 10.1007/S00432-006-0076-X

## Beschreibung

Die Erfindung betrifft ein Verfahren zur Abreicherung von zirkulierenden Tumorzellen aus einer Zellsuspension, wie es gattungsgemäß aus der WO 02/094350 A2 bekannt ist.

Aus dem Stand der Technik sind Verfahren bekannt, mit deren Hilfe eine Aufreinigung von Flüssigkeiten und Suspensionen möglich ist, die neben gesunden Zellen auch geschädigte, krankhaft veränderte und / oder blutfremde Zellen beinhalten. Dabei soll die Konzentration der geschädigten oder krankhaft veränderten Zellen so weit als möglich gesenkt werden, ohne die gesunden Zellen zu schädigen, bzw. es sollen nachteilige Wirkungen auf die gesunden Zellen so gering wie möglich gehalten werden. Verfahren zur Aufreinigung von Körperflüssigkeiten wie Blut oder Blutplasma außerhalb des Körpers werden auch als Apherese bezeichnet.

In WO 2009/155 384 A1 wird eine Methode und Vorrichtung für die selektive Entfernung von Zellen (u. a. Krebszellen), Krankheitserregern oder Viren durch die Anwendung von funktionalisierten superparamagnetischen Nanopartikeln beschrieben. Die Nanopartikel sind mit einem Bindungspartner funktionalisiert, der an die Oberfläche der Targetzellen, Krankheitserreger oder Viren andockt. Die Vorrichtung beinhaltet im extrakorporalen Kreislauf einen Magnetfilter, bestehend aus einem Magnet und einem wechselbaren magnetisierbaren Substrat (Netz o. ä.).

In WO 90/04019 A1 wird ein System für eine magnetisch affinite Zellseparation von Zellkonzentraten (heterogenen Zellmischungen) beschrieben. Die Zellkonzentrate werden dazu mit paramagnetischen µm-Partikeln, gecoatet mit Antikörpern oder anderen Bindungsagenzien für eine aktive Bindung zu den gewünschten Zellen (explizit Partikel/Zell-Konjugate) in Kontakt gebracht. Für die Separation wird ein 2-Magnetsystem zur Ausführung der Separation beschrieben.

Beide Patente beziehen sich auf spezifische Separationsverfahren. In der Diagnostik finden solche Verfahren bereits eine breite Anwendung. Einer therapeutischen Anwendung zur Entfernung von zirkulierenden Tumorzellen stehen allerdings bisher gravierende Nachteile entgegen. Einerseits ist die Ankopplung der Antikörper ein technisch kompliziertes Verfahren und andererseits bestehen Einschränkungen bezüglich einer quantitativen Markierung von Tumorzellen. Es gibt eine hohe Variabilität dieser Zellen und andererseits treten durch den biologischen Zelltransformationsprozess, Epithelial Mesenchymal Transition (EMT), Verluste der epithelialen Charakteristik auf, so dass diese Zellen durch Antikörper kaum markiert werden können. Des Weiteren ist die Herstellung der Antikörper ein teures Verfahren.

Aus der WO 02/094350 A2, der DE 101 27 068 A1 und dem Fachartikel "Differential interaction of magnetic nanoparticles with tumor cells and peripheral blood cells" von J. H. Clement et al. ist ein unspezifisches Verfahren bekannt, bei dem magnetische Nanoteilchen (fortan kurz: Nanopartikel) verwendet werden, um geschädigte Leukozyten oder blutfremde Zellen wie Tumorzellen aus einer abgetrennten Leukozytenfraktion so weit wie möglich zu entfernen. Dazu wird Blut mittels Apherese in die zellulären und nicht-zellulären Bestandteile fraktioniert und die zelluläre Fraktion in einen Arbeitspuffer überführt, wodurch eine Zellsuspension erhalten wird. Anschließend wird die Zellsuspension mit den magnetischen Nanopartikeln inkubiert. Die Partikelgrößen (fortan: mittlere Durchmesser) sollten dabei zwischen 2 und 100 nm liegen, wobei sehr vorteilhaft eine Mischung sehr kleiner und sehr großer Nanopartikel verwendet werden soll. Die Nanopartikel werden im Überschuss eingesetzt. Die Nanopartikel sollten mit einer biokompatiblen Schicht beschichtet sein. Während der Inkubationsdauer werden die Nanopartikel teils endozytotisch in die Zellen aufgenommen und / oder selektiv an deren Oberflächen gebunden. Dabei erfolgt die endozytotische Aufnahme bzw. das Binden an deren Oberfläche bei den gesunden Zellen weniger schnell als bei den krankhaft veränderten, geschädigten und blutfremden Zellen. Krankhaft veränderte und blutfremde Zelle können Tumorzellen sein, die einen wesentlich erhöhten Stoffwechsel aufweisen und daher unter anderem eine erhöhte endozytotische Aktivität zeigen. Bei einer zu langen Inkubationsdauer werden auch durch gesunde Zellen viele Nanopartikel aufgenommen bzw. es werden viele Nanopartikel an diese gebunden, wodurch anschließend auch diese gesunden Zellen aus der Zellsuspension abgetrennt werden würden. Die Inkubation muss nach der vorgesehenen Inkubationsdauer von maximal 8 Minuten zwingend mittels einer eiweißhaltigen Stopplösung abgebrochen werden. Nach dem Abstoppen der Inkubationsreaktion werden die verbliebenen Nanopartikel und die mit magnetischen Nanopartikeln beladenen Zellen aus der Zellsuspension abgetrennt, indem diese durch einen Magnetfilter gepresst wird.

Zur Durchführung des Verfahrens nach der vorgenannten WO 02/094350 A2 ist es erforderlich, dass die Anteile der gesunden Zellen sowie der Anteil der geschädigten, krankhaft veränderten und blutfremden Zellen bekannt sind. Da diese Anteile sehr spezifisch von der jeweiligen Zellsuspension abhängen, ist eine individuelle Modifikation der Verfahrensschritte erforderlich. Der Anteil der geschädigten, krankhaft veränderten und blutfremden Zellen wird durch das Verfahren gesenkt, was nachfolgend auch als Abreichern bezeichnet wird.

Der Erfindung liegt die Aufgabe zugrunde, eine weitere Möglichkeit zur Abreicherung von geschädigten, krankhaft veränderten und blutfremden Zellen vorzuschlagen, die effizient durchführbar ist und einen hohen Grad der Abreicherung erlaubt.

Die Aufgabe wird durch ein Verfahren zur Abreicherung von zirkulierenden Tumorzellen aus einer Zellsuspension gelöst, das die folgenden Schritte aufweist:
Zuerst wird eine Zellsuspension bereitgestellt, die eine Mischung von Zellen und abzureichernden Tumorzellen enthält. Dann erfolgt die Inkubation der Zellsuspension für eine Inkubationsdauer mit biokompatibel beschichteten magnetischen Nanopartikeln. Anschließend, nach Ablauf der Inkubationsdauer, wird ein magnetisches Abtrennen von mit magnetischen Nanopartikeln beladenen Zellen sowie von freien magnetischen Nanopartikeln aus der inkubierten Zellsuspension durchgeführt. Das erfindungsgemäße Verfahren ist dadurch gekennzeichnet, dass die Zellsuspension auf eine Inkubationstemperatur temperiert und während der Inkubation über die Inkubationsdauer auf der Inkubationstemperatur gehalten wird. Außerdem wird die Inkubation ohne Zugabe einer Stopplösung am Ende der Inkubationsdauer durchgeführt.

Bei dem erfindungsgemäßen Verfahren handelt es sich um ein unspezifisches magnetisches Separationsverfahren, bei dem die abzutrennenden geschädigten, krankhaft veränderten und / oder blutfremden Zellen ohne Verwendung von beispielsweise Antikörpern aus der Zellsuspension abgetrennt und damit abgereichert werden können.

Die Zellen liegen als eine mit einer geeigneten Pufferlösung versehene Zellsuspension vor. Eine geeignete Pufferlösung dient der Erhaltung der biologischen Aktivität der Zellen über die Dauer der Verfahrensdurchführung. Geeignete Pufferlösungen sind dem Fachmann bekannt.

Nachfolgend werden Zellen als mit Nanopartikeln beladene Zellen, oder kurz als beladene Zellen, bezeichnet, wenn diese Nanopartikel aufgenommen haben oder diese Zellen über ihre Oberflächen mit Nanopartikeln verbunden sind oder die Zellen sowohl aufgenommene Nanopartikel als auch Nanopartikel auf ihrer Oberfläche aufweisen. Tumorzellen sind krankhaft veränderte Zellen. Diese sind blutfremd, da sie im Blut eines gesunden Menschen im Allgemeinen nicht vorkommen.

Die Inkubationstemperatur beträgt mehr als 32°C, beispielsweise 32,5°C, 33°C, 34°C, 35°C, 36°C, 37°C oder 38°C

Das magnetische Abtrennen der freien biokompatibel beschichteten magnetischen Nanopartikel (auch kurz: Nanopartikel) sowie Zellen, die mit Nanopartikeln beladen sind (beladene Zellen), erfolgt entlang einer Trennstrecke. Dabei wird die inkubierte Zellsuspension entlang der Trennstrecke bewegt. Ist die Trennstrecke beispielsweise von flexiblen Wänden umfangen, beispielsweise von Wänden eines Beutels aus einem flexiblen Material wie Kunststoff, verhindert der durch die inkubierte Zellsuspension bewirkte statische Druck ein Kollabieren des Beutels. Durch geeignete Dimensionierung eines oder mehrerer Eingänge und Ausgänge des Beutels wird der statische Druck auf die Wände des Beutels erzeugt. Der Beutel ist in den Wirkungsbereich mindestens eines Magnetfeldes gebracht. Durch Wechselwirkungen der magnetischen Nanopartikel mit dem Magnetfeld werden Kräfte erzeugt, durch die freie Nanopartikel und beladene Zellen beeinflusst werden. Werden beispielsweise hinreichend große Anziehungskräfte zwischen den freien Nanopartikeln / den beladenen Zellen und dem Magnetfeld bewirkt, werden die freien Nanopartikeln / die beladenen Zellen abgelenkt und vorzugsweise an der Wand des Beutels gehalten (immobilisiert).

Es ist möglich, dass die inkubierte Zellsuspension entgegen der Schwerkraft entlang der Trennstrecke bewegt wird. Dabei wird die Aufrechterhaltung des statischen Drucks vorteilhaft unterstützt. In weiteren Ausgestaltungen des erfindungsgemäßen Verfahrens kann die inkubierte Zellsuspension auch horizontal entlang der Trennstrecke bewegt werden.

Es ist für das Verfahren essentiell, dass die magnetischen Nanopartikel mit Dextran oder Dextranderivaten, z. B. mit Carboxymethyldextran, beschichtet sind. Eine solche Beschichtung ist biokompatibel, sie ruft also in Wechselwirkung mit Zellen und Blutbestandteilen der Zellsuspension keine nachteiligen Reaktionen wie Immunreaktionen, beispielsweise Entzündungen oder gar Apoptose, hervor.

Die Nanopartikel werden vorzugsweise in Form eines Magnetofluids bereitgestellt, indem die biokompatibel beschichteten Nanopartikel in deionisiertem Wasser (DI-Wasser) dispergiert werden.

Die magnetischen Nanopartikel sind mit einem mittleren arithmetischen Durchmesser (Kerndurchmesser, ohne Beschichtung) aus einem Bereich von 20 bis 40 nm, insbesondere mit einem mittleren arithmetischen Durchmesser von 30 nm, gewählt. Werden Nanopartikel mit einem solchen mittleren Durchmesser verwendet, ergibt sich gegenüber dem Stand der Technik ein großer Vorteil. Es ist vorauszuschicken, dass die Inkubation der Zellsuspension und die Aufnahme von bzw. die Beladung der Zellen mit Nanopartikeln ein zeitabhängiger Vorgang ist. Vereinfachend kann angenommen werden, dass desto mehr beladene Zellen entstehen, je länger die Inkubation andauert. Dabei werden sehr kleine Nanopartikel, beispielsweise solche mit einem mittleren Durchmesser von 2 nm, sehr viel schneller aufgenommen als Nanopartikel mit einem mittleren Durchmesser von beispielsweise 100 nm. Die Aufnahme sehr kleiner, dann superparamagnetischer Nanopartikel erfolgt schnell, sodass lange Inkubationsdauern vermieden werden können. Allerdings besteht infolge der schnellen Aufnahme auch die Gefahr, dass auch die gesunden beladenen Zellen beim Abtrennen der mit Nanopartikeln beladenen Zellen zu einem erheblichen Anteil entfernt werden. Nanopartikel mit größeren Durchmessern von beispielsweise 100 nm werden deutlich langsamer aufgenommen und lassen entsprechend lange Inkubationsdauern erforderlich werden, um die Zellen zu beladen. Die Inkubation der Zellsuspension stellt auch für die gesunden Zellen eine physiologische Belastung dar, so dass überlange Inkubationsdauern von beispielsweise 30 Minuten bis zu mehreren Stunden zu vermeiden sind, um nicht den Großteil der gesunden Zellen zu verlieren.

Durch den Größenbereich von 20 bis 40 nm ist überraschenderweise ein Bereich optimaler mittlerer arithmetischer Durchmesser der Nanopartikel gefunden. Diese Nanopartikel sind klein genug, um effizient aufgenommen werden zu können, aber groß genug, dass die Aufnahme nicht in sehr kurzer Zeit erfolgt. Bei größeren (Nano-)Partikeln ist die Stabilität des Magnetofluids nicht gegeben, da die dispergierten Partikel aufgrund der Gravitation in kurzer Zeit sedimentieren und die Dispersion der Partikel aufgehoben wird. Zudem weisen Partikel mit einem größeren Durchmesser eine größere Remanenz auf.

Superparamagnetische Nanopartikel mit mittleren Durchmessern von 2 nm, wie diese in der vorgenannten WO 02/094350 A2 angegeben sind, sind aufgrund geringer Sättigungsmagnetisierung für das Abtrennen mittels Magnetfeldern nur bedingt geeignet. Andererseits bedingen der zellbiologische Prozess der Endozytose (Phagozytose / Pinozytose) für eine hohe Differenzierung zwischen gesunden Zellen (z. B. gesunden Leukozyten) und Tumorzellen in einem praktikablen Zeitrahmen von beispielsweise weniger als 20 Minuten, besser weniger als 15 Minuten, beispielsweise 8, 9 oder 10 Minuten, und die Stabilität des einzusetzenden Magnetofluids eine möglichst enge Teilchengrößenverteilung der Nanopartikel. Der Größenbereich 20 bis 40 nm ist dafür optimal geeignet.

Die Verwendung von Nanopartikeln mit einem mittleren Durchmesser ausgewählt aus dem Größenbereich von 20 bis 40 nm erlaubt vorteilhaft die Durchführung des erfindungsgemäßen Verfahrens, ohne dass die Inkubationsreaktion durch Zugabe einer Stopplösung abgebrochen werden muss. Das erfindungsgemäße Verfahren kann daher vorteilhaft während der Inkubation mit weniger Aufwand und unter Einsparung einer Komponente durchgeführt werden, als dies aus dem Stand der Technik bekannt ist. Zudem ist die Durchführung der Inkubation bei dem erfindungsgemäßen Verfahren bei Weitem nicht so zeitkritisch, wie dies im Stand der Technik ausdrücklich beschrieben ist. Dadurch ist das erfindungsgemäße Verfahren in der Praxis besser zu handhaben. Trotzdem ist natürlich für eine reproduzierbare Durchführbarkeit des erfindungsgemäßen Verfahrens die Einhaltung von entsprechenden Protokollen erforderlich.

Die Nanopartikel werden vorzugsweise aus dem vorgenannten Größenbereich ausgewählt. Der enge Teilchengrößenbereich erhöht die Reproduzierbarkeit der Verfahrensführung.

Das erfindungsgemäße Verfahren ist vorteilhaft mit Zellsuspensionen durchgeführt, die ein Leukozytenkonzentrat sind. Die Zellsuspension kann beispielsweise durch bekannte Aphereseverfahren bereitgestellt werden.

Für die Verfahrensdurchführung ist es bevorzugt, dass die Zellsuspension mit den magnetischen Nanopartikeln vor der Inkubation homogenisiert wird und die Zellsuspension während der Inkubationsdauer in Ruhe gehalten wird. Dadurch ist eine mechanisch bedingte Stresseinwirkung auf die Zellen in der Zellsuspension während der Inkubation stark reduziert.

Es ist vorteilhaft, wenn zum Abtrennen der mit magnetischen Nanopartikeln beladenen Zellen sowie von freien magnetischen Nanopartikeln die inkubierte Zellsuspension mit einer laminaren Strömung entlang der Trennstrecke bewegt wird. Durch eine laminare Strömung ist ebenfalls die Einwirkung von mechanischem Stress auf die Zellen gegenüber turbulenten Strömungen reduziert. Außerdem ist ein Abtrennen der beladenen Zellen und der freien Nanopartikel aus einer laminaren Strömung heraus effizienter als aus einer turbulenten Strömung, da weniger Strömungs- und Transportkräfte auftreten, die einer magnetisch bedingten Anziehungskraft entgegenwirken.

Zur Durchführung des erfindungsgemäßen Verfahrens wird sich ein vitaler zellbiologischer Prozess, die Endozytose, zunutze gemacht. Bei der Endozytose erfolgt ein Stoff- und Partikeltransport von außen durch die Zellmembran in das Zellinnere. Die Endozytose läuft bei Tumorzellen im Vergleich zu gesunden (Blut-)Zellen schneller ab. Durch die Inkubation der Zellsuspensionen, hier Tumorzellen enthaltende Leukozytenkonzentrate nach einer Blutauftrennung (Apherese) gemäß dem bekannten Stand der Technik, mit magnetischen Nanopartikeln eines mittleren Durchmessers von 20 bis 40 nm werden die gesunden Zellen und die abzureichernden Zellen voneinander abgetrennt. Die Nanopartikel sind vorzugsweise mit einer biokompatiblen Beschichtung versehen.

Zur Durchführung des erfindungsgemäßen Verfahrens wird die Zellsuspension mit einem Inkubationspuffer und dem Magnetofluid zu einem Separationsmedium vermischt. Der Inkubationspuffer kann beispielsweise aus PBS und EDTA bestehen.

Das Gemisch, in dem die Inkubation abläuft, enthält zudem mindestens 0,5% Protein. In weiteren Ausgestaltungen des Verfahrens kann der Proteingehalt mindestens 1,5%, mindestens 2,5%, mindestens 3,5% oder mindestens 4,5% oder 5% betragen.

Unter Einhaltung einer exakten Vorgehensweise (Protokoll) bei der Durchführung des erfindungsgemäßen Verfahrens wird eine mengenmäßig unterschiedliche Beladung der gesunden Zellen und der abzureichernden Zellen mit Nanopartikeln erreicht. Durch eine an die Inkubation anschließende Einwirkung eines Magnetfeldes ist eine Abtrennung der abzureichernden Zellen, beispielsweise von Tumorzellen, vom übergroßen Teil der gesunden Zellen möglich. Die Abtrennung erfolgt durch ein Fließverfahren, bei dem die Zellsuspension mit Hilfe einer Pumpe durch einen Magnetseparator gepumpt wird. Das eigentliche Wertprodukt der Separation ist das Eluat, das die vom Magnetseparator nicht zurückgehaltenen gesunden Zellen enthält. Durch diese schonende externe Zellabtrennung bleibt die Vitalität der gesunden Zellen weitestgehend erhalten. Wird die aufgereinigte Fraktion (Eluat) nach der Durchführung des erfindungsgemäßen Verfahrens beispielsweise wieder einem Patienten zugeführt, kann aufgrund der weitestgehend unbeeinträchtigten gesunden Zellen die Immunabwehr des Patienten unterstützt werden.

Nanopartikel können wie nachfolgend beschrieben hergestellt werden. Das Verfahren betrifft die Herstellung von auf Magnetit basierenden Nanopartikeln in einem Größenbereich von 20 bis 40 nm.

Magnetische Nanopartikel haben eine ständig wachsende Bedeutung in der Biomedizin, vor allem aufgrund neuer sehr aussichtsreicher Applikationsfelder. Das betrifft u. a. die gezielte Medikamentenfreisetzung (Drug Delivery / Drug Targeting), neue Kontrastagenzien für MRI, Magnetpartikelbildgebung (MPI), Krebstherapie unter Anwendung der Magnetofluid-Hyperthermie (MFI), magnetische Zellseparationen und Anwendungen als Nano-/Biosensoren.

Prinzipiell kommen für die Herstellung von magnetischen Nanopartikeln zahlreiche Verfahren zur Anwendung. Zu den chemischen Verfahren gehören nasschemische Fällungsverfahren, hydrothermale Umwandlungen und Glaskristallisationsverfahren. Zu den physikalischen Verfahren gehören Hochtemperaturzersetzungen, Aerosolverfahren, Kondensation von Metalldampf und Laserpyrolyseverfahren.

Monodisperse Nanopartikel auf der Basis von Magnetit (Magnetitnanopartikel) werden vor allem über organische Synthesewege hergestellt. Die Herstellbarkeit stabiler wässriger Dispersionen als Voraussetzung für biomedizinische Anwendungen ist dadurch allerdings sehr begrenzt. Für einen Phasentransfer sind umfangreiche Manipulationen (z. B. über amphiphile Polymere) notwendig und für Routineverfahren schwer praktikabel.

Für biomedizinische Anwendungen werden deshalb nasschemische Herstellungsverfahren favorisiert. Dabei liegt ein Fokus auf Magnetitnanopartikeln, die für biomedizinische Anwendungen zugelassen sind.

Zur deren Herstellung auf nasschemischem Wege gibt es prinzipiell zwei Hauptmethoden:
1. Fällung einer stark alkalischen Mischung von Fe(II)- und Fe(III)-Salzen im stöchiometrischen Verhältnis: Das Verfahren, erstmals von Massart [Massart et al.: J. Chem. Phys. 84 (1987) 767] erwähnt, wurde hinsichtlich der Verfahrensparameter in zahlreichen Veröffentlichungen untersucht und modifiziert u. a. von Jolivet und Vayssieres [Jolivet et al.: J. of Colloid and Interface Science 205 (1998) 205].
2. Partielle Oxidation von Fe(II)-Salzen unter Anwendung unterschiedlicher Oxidationsmittel: Das Verfahren wurde beispielsweise von Sugimoto und Matijevic beschrieben [Sugimoto et al.: J. of Colloid and Interface Science 74 (1980) 1, 227].

Die Teilchengrößen der Nanopartikel, hergestellt nach o. g. Verfahren, unterscheiden sich stark. Nach dem Herstellungsverfahren 1 (Massart-Verfahren) erzielt man Nanopartikel mit mittleren Durchmessern in einem Bereich von 5 bis 15 nm, d.h. Nanopartikel mit superparamagnetischen Eigenschaften. Nach dem Verfahren der partiellen Oxidation (Verfahren 2) beträgt die Nanopartikelgröße 30 bis 200 nm. Im Allgemeinen erhält man nach letzterem Verfahren eine relativ breite Partikelgrößenverteilung.

Für biomedizinische Anwendungen finden weitestgehend Modifikationen des Massart-Verfahrens Anwendung, wie dies beispielsweise in den Schriften US 5 262 176 A ("Synthesis of polysaccharide covered superparamagnetic oxide colloids"), DE 34 43 252 A1 ("Dextran-Magnetit-Komplexe für die NMR-Diagnostik"), WO 94/21240 A2 ("Superparamagnetische Teilchen, Verfahren zu ihrer Herstellung und Verwendung"), US 4 554 088 A ("Magnetic particles for use in separations"), DE 44 28 851 A1 ("Eisen enthaltende Nanopartikel, ihre Herstellung und Anwendung in der Diagnostik und Therapie") und US 4 101 435 A ("Magnetic Iron oxide-dextran complex and process for its production") beschrieben ist.

Als Stand der Technik für Verfahren zur Herstellung von Magnetitnanopartikeln in einem Größenbereich von 20 bis 40 nm sind nachfolgend beispielhaft einige angeführt.

Verges et al. (J. Phys. D: Appl. Phys. 41 (2008) 134003) beschreiben ein modifiziertes Verfahren der Nanopartikelherstellung, bei der die Fällung hierbei bei Raumtemperatur und anschließender Agingphase bei 90°C erfolgt. Diese Vorgehensweise erfordert einen sehr langen Zeitumfang von 24h. Die Herstellung von Nanopartikeln mit einer mittleren Teilchengröße von etwa 30 nm ist bei diesem Verfahren nur im Reaktionsmedium H₂O/EtOH möglich.

Santoyo-Salazar et al. offenbaren die Herstellung von Magnetitnanopartikeln im Bereich 20 bis 40 nm durch Hydrolyse von FeCl₂ mit KOH bei 50°C unter Zusatz von kochendem DI-Wasser (Materials Science and Engineering C 27 (2007) 1317). Konkrete Angaben zum Verfahren werden nicht genannt.

Weiterhin ist durch Wen-Guang et al. (Materials Science and Engineering B 136 (2007) 101) die Herstellung von Nanopartikeln auf der Basis von Magnetit durch partielle Oxidation von FeSO₄x7H₂O beschrieben. Bei diesem Verfahren wird das alkalische Medium (NaOH) bei Temperaturen von 60 bis 100°C vorgelegt und das Fe(II)-Salz langsam dazudotiert. Erst im nächsten Schritt erfolgt langsam der Zusatz des Oxidationsmittels NaNO₃. Nach diesem Verfahren sind die kleinsten Nanopartikel mit einem Durchmesser von 34 nm bei 60°C und in einer Reaktionszeit von 4h herstellbar. Allerdings müssen dabei niedrige Fe(II)-Salzkonzentrationen (0,2 mol/l) angewandt werden, wobei eine Patina als Zwischenschicht entsteht.

Nishio et al. geben eine langsame Oxidation von Fe(II)-Salzen mit Natriumnitrat in einem stark alkalischen Medium (pH12) bei Temperaturen von 4°C bis maximal 40°C an (Journal of Magnetism and Materials 310 (2007) 2408). Die Reaktion verläuft sehr langsam über die Stadien der Bildung von Grünem Rost, Lepidokrokit und Goethit und erfordert für eine reproduzierbare Herstellung von Magnetit hohe Anforderungen bezüglich der inerten Versuchsbedingungen. Außerdem können mit diesem Verfahren effektiv nur sehr geringe Mengen an Magnetitnanopartikeln hergestellt werden.

Es wird daher hier eine Möglichkeit zur Herstellung von magnetischen Nanopartikeln insbesondere mit einem mittleren Durchmesser in einem Bereich von 20 bis 40 nm vorgeschlagen.

Das vorgeschlagene Herstellungsverfahren geht von einem Zusatz von Fe(II)-Salzen zu einem 70 bis 80°C temperierten N₂-gespülten Reaktionsbad aus. Die Fällung und partielle Oxidation der Fe-Hydroxide erfolgt durch langsame automatische Zudosierung eines Base/Oxidationsmittel-Gemisches. Es erfolgt eine in situ pH-Messung und - Steuerung während der Reaktions- und Alterungsphase. Unmittelbar nach der Dosierung des Oxidationsmittel / Hydroxid-Gemisches wird eine pH-Änderung (von pH5 auf pH11) durch potentiometrische Titration erzwungen und der pH-Wert während der Alterungsphase konstant gehalten.

Die Abtrennung der freien magnetischen Nanopartikel sowie der mit magnetischen Nanopartikeln beladenen Zellen aus einer inkubierten Zellsuspension, enthaltend eine Mischung von Zellen und abzureichernden Tumorzellen, freien magnetischen Nanopartikeln sowie von mit magnetischen Nanopartikeln beladenen Zellen erfolgt mit einer entsprechenden Vorrichtung. Die Vorrichtung weist dabei beispielsweise einen durchströmbaren Beutel auf, der mindestens einen Eingang zum Einströmen der inkubierten Zellsuspension in einen Innenraum des Beutels aufweist. Außerdem weist der Beutel mindestens einen Ausgang zum Ausströmen der inkubierten Zellsuspension aus dem Innenraum des Beutels auf. Dabei ist die Strecke zwischen dem Eingang und dem Ausgang eine Trennstrecke zum magnetischen Abtrennen der freien magnetischen Nanopartikel sowie der mit magnetischen Nanopartikeln verbundenen Zellen und Tumorzellen. Die Vorrichtung weist außerdem mindestens einen Magneten auf, der neben der Trennstrecke so angeordnet ist, dass Magnetfeldlinien des Magneten den Innenraum des Beutels durchdringen, sodass die mit magnetischen Nanopartikeln beladenen Zellen sowie die freien magnetischen Nanopartikel zu mindestens einer Wand des Beutels hin abgelenkt und dort gehalten werden.

In weiteren Ausführungen einer Vorrichtung können auch mehrere Eingänge und mehrere Ausgänge vorhanden sein.

Der mindestens eine Magnet kann ein Permanentmagnet sein. In einer weiteren Ausführung der Vorrichtung ist der mindestens eine Magnet ein Elektromagnet. Der Elektromagnet ist vorzugsweise mit einer Steuerungseinheit zur Steuerung des Elektromagneten verbunden. Durch die Steuerungseinheit kann auch zusätzlich oder alternativ die Pumpe gesteuert sein.

Es können anstatt des mindestens einen Magneten oder zusätzlich zu diesem auch Magnetanordnungen vorhanden sein, in denen mehrere Magnete angeordnet sind.

Die Vorrichtung kann vorteilhaft zum Abtrennen von magnetischen Körpern aus einem flüssigen Medium verwendet werden. Dabei erfolgt das Abtrennen, während das Medium den Innenraum des Beutels durchströmt. Das Medium ist beispielsweise eine Zellsuspension, die mit magnetischen Nanopartikeln inkubiert wurde. Die Nanopartikel weisen einen mittleren Durchmesser aus einem Bereich von 20 bis 40 nm auf.

Wird die Vorrichtung zum Abtrennen von magnetischen Körpern aus einem flüssigen Medium verwendet, ist es günstig, wenn das Medium den Innenraum mit einer laminaren Strömung durchströmt. Dadurch sind Einwirkungen mechanischen Stresses auf die Zellen in der Zellsuspension vorteilhaft reduziert.

Ein laminares Strömungsprofil des Mediums beim Durchströmen des Innenraums des Beutels kann beispielsweise erreicht werden, indem ein flacher bis ovaler Querschnitt des Innenraums des Beutels gewählt wird und sowohl die Eingänge als auch die Ausgänge nebeneinander in der Längsrichtung des Querschnitts angeordnet sind. Es ist außerdem möglich, dass sich jeweils ein Eingang und ein Ausgang gegenüberliegen. Zwischen Einmündungen der jeweils äußersten Eingänge bzw. den Einmündungen der jeweils äußersten Ausgänge und einer Wand des Beutels ist vorzugsweise nur ein so großer Abstand vorhanden, dass dort keine turbulenten Strömungen, beispielsweise durch die Ausbildung von Rückströmungen, entstehen können bzw. diese auf ein geringes Maß beschränkt sind.

Die Erfindung wird nachfolgend anhand eines Ausführungsbeispiels näher erläutert. Dabei zeigt:

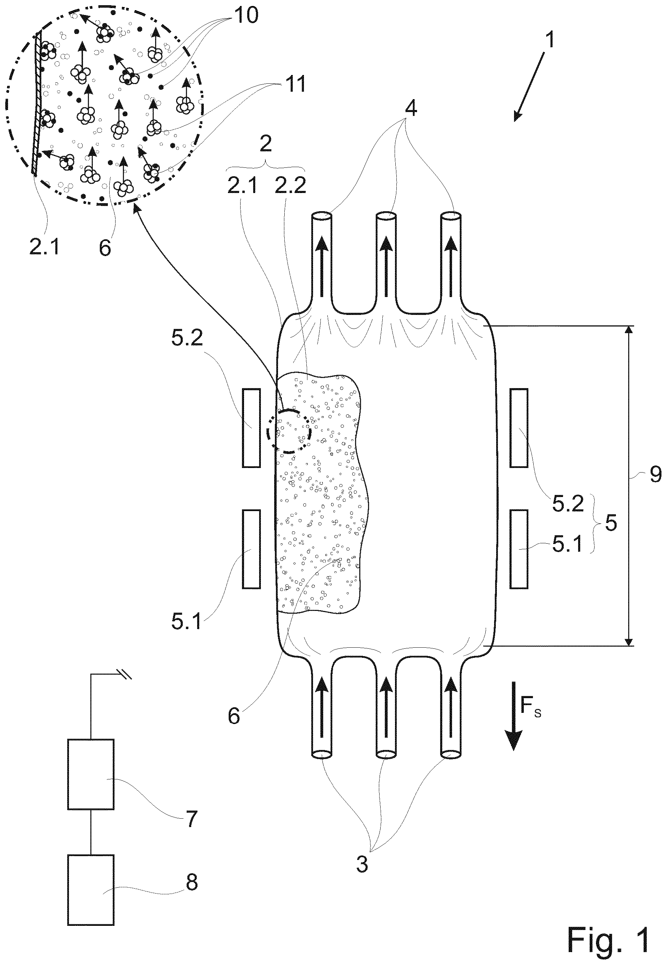

- Fig. 1: ein Ausführungsbeispiel einer Vorrichtung zum Abtrennen beladener Zellen und freier magnetischer Nanopartikel aus einem Medium.

Für die Durchführung des erfindungsgemäßen Verfahrens wird zunächst die inkubierte Zellsuspension wie folgt hergestellt: Die Zellsuspension ist ein Leukozytenkonzentrat in einem Puffer. Zur Inkubation wird ein Separationsmedium bereitgestellt, das aus PBS (phosphatgepufferte Salzlösung) und 2 mmol EDTA besteht. Es wird zudem das Magnetofluid, bestehend aus DI-Wasser und darin dispergierten biokompatibel beschichteten Nanopartikeln 10 mit einem mittleren arithmetischen Durchmesser von 30 nm, die Zellsuspension und Plasma zugefügt. Der resultierende Proteingehalt des Separationsmediums beträgt mindestens 0,5%. Nach einer anfänglichen Durchmischung der Bestandteile des Separationsmediums wird die Inkubation durchgeführt, indem das Separationsmedium über die Inkubationsdauer von mindestens 8 und höchstens 12 Minuten weder gerührt, noch geschüttelt, noch in sonstiger Weise bewegt wird. Die Zellsuspension wird auf eine Inkubationstemperatur von mehr als 32°C, aber nicht mehr als 38°C, temperiert und über die Inkubationsdauer auf der Inkubationstemperatur gehalten. Während der Inkubation wird ein Anteil der Nanopartikel 10 durch die Zellen 11 der Zellsuspension endozytotisch aufgenommen und / oder diese heften sich an die Oberflächen der Zellen 11. Die endozytotische Aufnahme erfolgt bei Tumorzellen schneller als bei gesunden Zellen 11. Über die Inkubationsdauer werden daher in Tumorzellen mehr Nanopartikel 10 angereichert als in gesunden Zellen 11. Die Tumorzellen sind also stärker mit Nanopartikeln 10 beladen als die gesunden Zellen 11.

Nach Ablauf der Inkubationsdauer wird die inkubierte Zellsuspension dem Magnetseparator 1 zugeführt und strömt durch diesen hindurch.

Der Magnetseparator 1, gemäß Fig. 1, weist als wesentliche Elemente einen Beutel 2 mit mehreren Eingängen 3 und mehreren Ausgängen 4 sowie wenigstens eine Magnetanordnung 5 mit wenigstens einem ersten Magnet 5.1 auf.

In dem ersten Ausführungsbeispiel sind in Fig. 1 zwei Magnetanordnungen 5 gezeigt, die jeweils einen ersten Magneten 5.1 und einen zweiten Magneten 5.2 aufweisen. Eine der Magnetanordnungen 5 ist links des Beutels 2 und eine der Magnetanordnungen 5 ist rechts des Beutels 2 angeordnet. Die Eingänge 3 münden von unten in den Beutel 2 ein, während die Ausgänge 4 oben an dem Beutel 2 vorhanden sind.

Durch die Eingänge 3 strömt ein Medium 6, das eine mit magnetischen Nanopartikeln inkubierte Zellsuspension ist, in einen Innenraum 2.2 des Beutels 2 ein und tritt nach dem Durchströmen des Innenraums 2.2 durch die Ausgänge 4 wieder aus dem Beutel 2 aus. Das Medium 6 strömt auf seinem Weg durch den Innenraum 2.2 zwischen den beiden Magnetanordnungen 5 durch und wird durch deren Magnetfelder (nicht gezeigt) durchdrungen. Bei einem Betrieb des Magnetseparators 1 ist der Innenraum 2.2 durch das Medium 6 von unten nach oben durchströmt. Das Medium 6 strömt also entgegen der auch auf das Medium 6 wirkenden Schwerkraft F_{S} (durch einen Pfeil veranschaulicht).

Aufgrund des Vorhandenseins der Eingänge 3 und der Ausgänge 4 ist der Innenraum 2.2 entlang einer Trennstrecke 9 durch das Medium 6 mit einer laminaren Strömung durchflossen. Das Medium 6 wird mittels einer Pumpe 8 gefördert. Durch eine Steuerungseinheit 7 ist die Pumpe 8 steuerbar.

In dem Medium 6 sind freie magnetische Nanopartikel 10 sowie Zellen 11 vorhanden, die mit magnetischen Nanopartikeln 10 beladen sein können. Durchströmt das Medium 6 den Innenraum 2.2, treten die freien Nanopartikel 10 und die Nanopartikel 10 in den beladenen Zellen 11 mit den Magnetfeldern der Magnetanordnungen 5 in Wechselwirkung. Durch magnetisch bedingte Anziehungskräfte werden die freien Nanopartikel 10 und die Nanopartikel 10 in den beladenen Zellen 11 (als kugelige Konglomerate dargestellt) zu einer seitlichen Wand 2.1 des Beutels 2 abgelenkt. Da an der Wand 2.1 die Wechselwirkungen und Anziehungskräfte am stärksten sind, werden die freien Nanopartikel 10 und die beladenen Zellen 11 an der Wand 2.1 gehalten, wie dies in der Ausschnittsvergrößerung schematisch gezeigt ist. Die Pfeile an den Zellen 11 symbolisieren die Richtung, in die sich die Nanopartikel 10 bzw. die beladenen Zellen 11 durch ein Zusammenwirken der Anziehungskräfte und der Strömung in dem Beutel 2 bewegen. Unbeladene Zellen 11 bewegen sich in Richtung der Strömung, während Nanopartikel 10 und mit Nanopartikeln 10 beladene Zellen 11 in Richtung auf die Wand 2.1 abgelenkt werden.

Das restliche Medium 6 sowie Zellen 11, in denen keine Nanopartikel 10 vorhanden sind oder deren Beladung mit Nanopartikeln 10 so gering ist, dass die Wechselwirkungen mit den Magnetfeldern der Magnetanordnungen 5 keine zur Ablenkung und zum Halten der freien Nanopartikel 10 bzw. der beladenen Zellen 11 ausreichenden Anziehungskräfte bewirken, werden über den Ausgang 4 abgeführt.

### Bezugszeichenliste

- 1: Magnetseparator
- 2: Beutel
- 2.1: Wand
- 2.2: Innenraum
- 3: Eingang
- 4: Ausgang
- 5: Magnetanordnung
- 5.1: erster Magnet
- 5.2: zweiter Magnet
- 6: Medium
- 7: Steuerungseinheit
- 8: Pumpe
- 9: Trennstrecke
- 10: Nanopartikel
- 11: Zelle
- Fₛ: Schwerkraft

## Patentansprüche

1. Verfahren zur Abreicherung von zirkulierenden Tumorzellen aus einer Zellsuspension mit den Schritten:
- Inkubation einer bereitgestellten Zellsuspension, enthaltend eine Mischung von Zellen (11) und abzureichernden Tumorzellen, für eine Inkubationsdauer mit biokompatibel beschichteten magnetischen Nanopartikeln (10) und
- nach Ablauf der Inkubationsdauer magnetisches Abtrennen von mit magnetischen Nanopartikeln (10) beladenen Zellen (11) sowie von freien magnetischen Nanopartikeln (10) aus der inkubierten Zellsuspension,
**dadurch gekennzeichnet, dass**
- die Zellsuspension auf eine Inkubationstemperatur größer 32°C temperiert und während der Inkubation über die Inkubationsdauer von weniger 20 Minuten auf der Inkubationstemperatur gehalten wird,
- die verwendeten magnetischen Nanopartikel (10) einen mittleren Durchmesser von 20 bis 40 nm aufweisen und mit Dextran, Dextranderivaten oder Carboxymethyldextran beschichtet sind und
- die Inkubation ohne Zugabe einer Stopplösung am Ende der Inkubationsdauer durchgeführt wird.

2. Verfahren nach Anspruch 1, **dadurch gekennzeichnet, dass** das Abtrennen von mit magnetischen Nanopartikeln (10) beladenen Zellen (11) sowie der freien magnetischen Nanopartikel (10) entlang einer Trennstrecke (9) erfolgt, wobei die inkubierte Zellsuspension entgegen der Schwerkraft (F_{S}) entlang der Trennstrecke (9) bewegt wird.

3. Verfahren nach Anspruch 1 oder 2, **dadurch gekennzeichnet, dass** die Inkubationstemperatur nicht mehr als 38°C beträgt.

4. Verfahren nach einem der Ansprüche 1 bis 3, **dadurch gekennzeichnet, dass** die Inkubationsdauer mindestens 8 Minuten beträgt.

5. Verfahren nach einem der Ansprüche 1 bis 4, **dadurch gekennzeichnet, dass** die Zellsuspension ein Leukozytenkonzentrat ist.

6. Verfahren nach einem der vorhergehenden Ansprüche, **dadurch gekennzeichnet, dass** die Zellsuspension mit den magnetischen Nanopartikeln (10) vor der Inkubation homogenisiert wird und die Zellsuspension während der Inkubationsdauer in Ruhe gehalten wird.

7. Verfahren nach einem der vorhergehenden Ansprüche, **dadurch gekennzeichnet, dass** zum Abtrennen der mit magnetischen Nanopartikeln (10) beladenen Zellen (11) sowie der freien magnetischen Nanopartikel (10) die inkubierte Zellsuspension mit einer laminaren Strömung entlang der Trennstrecke (9) bewegt wird.

## Claims

1. Method for the removal of circulating tumor cells from a cell suspension, comprising the steps:
- a cell suspension provided, containing a mixture of cells (11) and tumor cells to be removed, is incubated for an incubation period with biocompatibly coated magnetic nanoparticles (10) and
- after the expiration of the incubation period, cells (11) loaded with magnetic nanoparticles (10) and free magnetic nanoparticles (10) are magnetically removed from the incubated cell suspension,
**characterized in that**
- the cell suspension is adjusted to an incubation temperature higher than 32°C and is kept at the incubation temperature during the incubation over the incubation period of less than 20 minutes,
- the magnetic nanoparticles (10) used have an average diameter of 20 to 40 nm and are coated with dextran, derivates of dextran or carboxymethyldextran and
- the incubation is carried out without addition of a stop solution at the end of the incubation period.

2. Method according to claim 1, **characterized in that** cells (11) loaded with magnetic nanoparticles (10) and the free magnetic nanoparticles (10) are removed along a removal section (9), wherein the incubated cell suspension is moved along the removal section (9) against the force of gravity (F_{S}).

3. Method according to claim 1 or 2, **characterized in that** the incubation temperature does not exceed 38 °C.

4. Method according to one of claims 1 to 3, **characterized in that** the incubation period is at least 8 minutes.

5. Method according to one of claims 1 to 4, **characterized in that** the cell suspension is a concentrate of leukocytes.

6. Method according to one of the preceding claims, **characterized in that** the cell suspension with the magnetic nanoparticles (10) is homogenized before the incubation and the cell suspension is kept at rest during the incubation period.

7. Method according to one of the preceding claims, **characterized in that** the incubated cell suspension is moved along the removal section (9) with a laminar flow for removal of the cells (11) loaded with magnetic nanoparticles (10) and of the free magnetic nanoparticles (10).

## Revendications

1. Procédé d'appauvrissement de cellules tumorales circulantes à partir d'une suspension de cellules, comprenant les étapes:
- une suspension de cellules fournie, contenant un mélange de cellules (11) et de cellules tumorales à appauvrir, est incubée pour une période d'incubation avec des nanoparticules (10) magnétiques revêtues de manière à être biocompatible,
- après l'expiration de la période d'incubation, des cellules (11) chargées de nanoparticules (10) magnétiques et des nanoparticules (10) magnétiques libres sont séparées de manière magnétique à partir de la suspension de cellules incubée,
**caractérisé en ce que**
- la suspension de cellules est ajustée à une température d'incubation supérieure à 32°C et est maintenue à la température d'incubation pendant l'incubation pendant la période d'incubation de moins de 20 minutes,
- les nanoparticules (10) magnétiques utilisées ont un diamètre moyen de 20 à 40 nm et sont revêtues de dextrane, de dérivés de dextrane ou de carboxyméthyldextrane et
- l'incubation est effectuée sans ajout d'une solution d'arrêt à la fin de la période d'incubation.

2. Procédé selon la revendication 1, **caractérisé en ce que** des cellules (11) chargées de nanoparticules (10) magnétiques et les nanoparticules (10) magnétiques libres sont séparées le long d'une section de séparation (9), la suspension de cellules incubée étant déplacée le long de la section de séparation (9) contrairement à la force de pesanteur (F_{S}).

3. Procédé selon la revendication 1 ou 2, **caractérisé en ce que** la température d'incubation ne dépasse pas 38°C.

4. Procédé selon une des revendications 1 à 3, **caractérisé en ce que** la période d'incubation est au moins 8 minutes.

5. Procédé selon une des revendications 1 à 4, **caractérisé en ce que** la suspension de cellules est un concentré de leucocytes.

6. Procédé selon une des revendications précédentes, **caractérisé en ce que** la suspension de cellules avec les nanoparticules (10) magnétiques est homogénéisée avant l'incubation et la suspension de cellules est maintenue au repos pendant la période d'incubation.

7. Procédé selon une des revendications précédentes, **caractérisé en ce que** la suspension de cellules incubée est déplacée le long de la section de séparation (9) avec un écoulement laminaire pour séparer les cellules (11) chargées de nanoparticules (10) magnétiques et les nanoparticules (10) magnétiques libres.
